# EUROPEAN PATENT SPECIFICATION

(11) **EP 1 603 473 B1**
(45) Date of publication and mention of the grant of the patent: **02.03.2016**
(21) Application number: 04717535.1
(22) Date of filing: 05.03.2004
(51) Int. Cl.: A61B 18/14

(54) **ELECTRICAL NEEDLE WITH RADIOPAQUE MARKER**
ELEKTRISCHE NADEL MIT RADIOLOGISCHEM MARKER
AIGUILLE ELECTRIQUE COMPRENANT UN MARQUEUR RADIOPAQUE

(30) Priority: 07.03.2003 US 382836
(43) Date of publication of application: 14.12.2005
(73) Proprietor: Avent, Inc., Alpharetta, GA 30004 (US)
(72) Inventor: KRISHNAMURTHY, Subashini, Toronto, Ontario M6P 2P4 (CA); SHAH, Krishan, Mississauga, Ontario L5M 2C7 (CA)
(74) Representative: Mabey, Katherine Frances
(86) International application number: PCT/CA2004/000338
(87) International publication number: WO 2004/078052

(56) References cited:
- WO-A1-02/089686
- WO-A2-02/054941
- US-A- 5 429 597
- US-A- 6 056 743
- US-A- 6 146 380
- US-A1- 2002 193 781
- US-A1- 2003 023 239
- US-A1- 2003 032 936

## Description

The invention relates to a needle that delivers electrical current and more particularly to a needle that delivers high frequency electrical current in the vicinity of a neural structure.

### BACKGROUND OF THE ART

A minimally invasive technique of delivering high frequency electrical current has shown to relieve localized pain in many patients. The high frequency electrical current is typically delivered from a generator via connected electrodes that are placed in a patient's body. The needles include an insulated shaft with an exposed electrically conductive tip. Tissue resistance to the high frequency electrical current at the tip causes heating of adjacent tissue. When temperature increases sufficiently tissue coagulates. The temperature that is sufficient to coagulate unmyelinated nerve structures is 45 °C, at which point a lesion is formed and pain signals are blocked. This results in relief from pain.

Needles with varying geometries are used in such applications. For example, the exposed tip of the needle can be pointed, blunt and rounded or open, varying in shape in accordance with the needs of different procedures. Pointed tips are self-penetrating while rounded tips are useful in soft tissue areas such as the brain where it is critical not to damage nerves. However, blunt needles can do more tissue damage than small diameter sharp needles. Open tips can be used to deliver a therapeutic agent during electrical treatment. United States Patent No. 6,146,380 to Racz et al. describes electrical needles with curved tips used in high frequency lesioning.

This technique of relieving back pain has also been used with needles penetrating the intervertebral disk. United States Patent Nos. 5,433,739 and 5,571,147 to Sluijter et al. and WIPO publication WO 01/45579 to Finch et al. describe needles that are used in the intervertebral disk to relieve back pain caused by herniated disks.

For treatment, the needle having a hollow shaft and a removable stylet therein is inserted into the patient's body and positioned. Once the needle is positioned, the stylet is withdrawn and a distal end of a high frequency probe is inserted until the distal end of the probe is at least flush with the distal end of the shaft, (i.e. the exposed tip). The probe is connected to an external signal generator that generates high frequency electrical current.

These needles are often used to denervate certain portions of a spine of the patient. Accurate placement of the needle in a complicated structure like the spine requires great technical skill by a treating clinician. In these procedures, the needle is viewed via X-ray or a fluoroscope to assist placement and is guided into the body. One limitation of the technique used currently is that the insulated shaft is not distinguishable from the exposed tip of the needle under X-ray or fluoroscopy. Therefore, accurate visualization of the exposed tip is not possible.

Prior art devices for accurate placement have not been used in conjunction with radio frequency needles. Radiopaque marking has been used to accomplish precise placement of catheters and stents. United States Patent No. 5,429,597 to Demello et al. discloses a balloon catheter having a radiopaque distal tip composed of a polymer mixed with a radiopaque powder such as tungsten. United States Patent No. 6,315,790 to Gerberding et al. describes a catheter constructed with radiopaque polymer hubs where the hubs provided the dual function of stent crimping and marker bands.

An example of a catheter utilizing an external marker band is described in United States Patent No. 5,759,174 to Fischell et al. The catheter has a single external metal marker band to identify the central portion of the stenosis once the delivery catheter is removed.

In spite of the improved illumination of the aforementioned devices when marked, there are some limitations to their application. Upon attachment conventional radiopaque markers may project from the surface of the catheter or stent, thereby causing a departure from its ideal profile. Some markers add rigidity to the stent and catheter in areas that had been designated for deformation. A needle for delivering radio frequency that overcomes some or all of the limitations of the prior art is desired.

WO 02/054941 discloses a needle with the features of the preamble of claim 1. Another needle is disclosed in WO 02/089686.

### SUMMARY OF THE INVENTION

In accordance with the present invention, there is provided a needle for insertion into a patient's body for delivering high frequency electrical current as set forth in claim 1.

The present invention provides for improved placement of a needle delivering high frequency energy by incorporating a radiopaque marker to distinguish the exposed tip from the shaft under fluoroscopic visualization.

To facilitate precise placement of the exposed tip, the tip is distinguishable from the rest of the needle when viewed under X-rays and fluoroscopy. When a needle with radiopaque marking, according to the present invention, is inserted in the patient's body, the location of a lesion made or to be made by the needle can be easily determined, as the tip of the needle can be distinguished from the electrically insulated shaft.

The present invention provides a needle for insertion into a patient's body comprising an electrically insulated shaft having an electrically conductive tip portion and a radiopaque marker.

The tip portion of the needle is the exposed tip and can be of varying dimensions. The radiopaque marker distinguishes the electrically insulated portion of the needle from the tip portion. This effectively identifies the position of the needle when in the body. The marker may be adapted to needles having various geometric shapes. The insulated portion of the needle includes an insulating coating. The coating may cover the radiopaque marker on the needle preventing a departure from the needle's true profile.

The radiopaque marker is band-shaped and can comprise radiopaque coatings of metals/polymers, or radiopaque materials deposited on the surface of the needle by techniques such as ion implantation or vapor deposition. These features and others will be apparent in the detailed description that follows.

### BRIEF DESCRIPTION OF THE DRAWINGS

In order that the invention may be readily understood, embodiments of the invention and arrangements not in accordance with the invention are illustrated by way of examples in the accompanying drawings, in which:
Fig. 1 is a schematic illustration of a needle connected to a high frequency generator, in accordance with an embodiment;
Fig. 2 is a side elevation view of an embodiment of the needle including a stylet;
Figures 3, 4, 5, 6 and 7 illustrate side elevation views of different configurations of a needle with radiopaque marking, wherein Figures 3 and 7 illustrate embodiments of a needle with radiopaque marking and
Figures 4, 5 and 6 illustrate alternative needles outside the scope of the present invention;
Figures 8, 9A and 9B illustrate modification to a stylet to impart radiopacity.

### DETAILED DESCRIPTION OF THE INVENTION

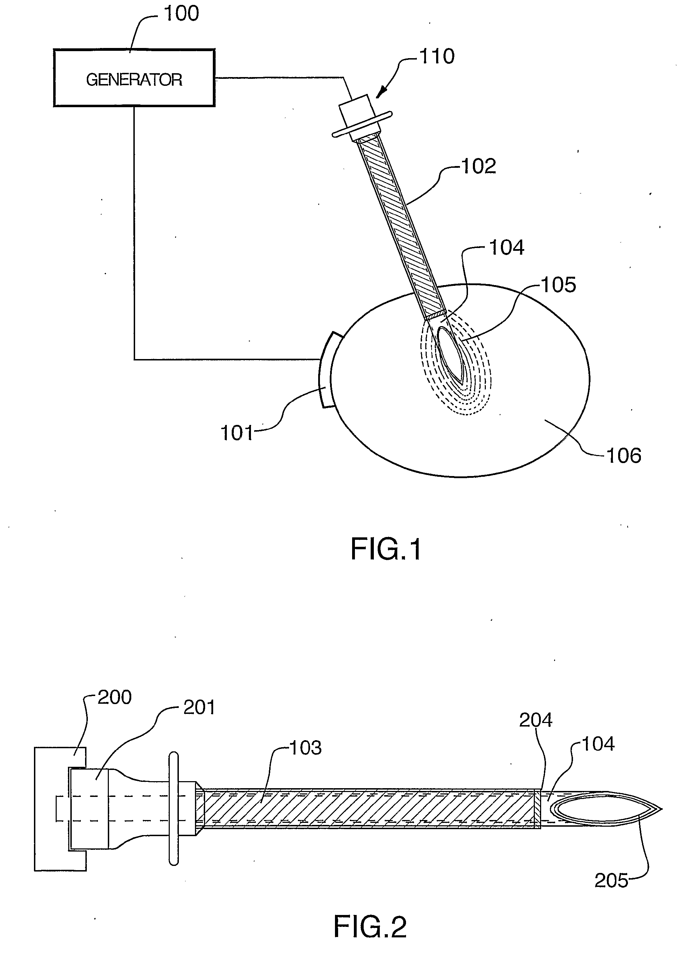

In accordance with an aspect of the invention a medical apparatus is provided for delivering high frequency electrical current to neural structures. As illustrated in Figure 1, the medical apparatus comprises a generator 100 for producing high frequency electrical current, a needle 102 with an electrical probe 110 connected to the generator 100 that is placed in the needle 102 for delivering the high frequency electrical current and a reference electrode 101 that completes the circuit. The needle 102 with the probe 110 is placed in a portion of a patient's body indicated generally at 106.

As can be seen more clearly in Figure 2, the hollow shaft of the needle 102 is covered with an insulating coating 103 leaving a portion of the tip 104 uncoated, exposed and electrically conductive. The tip 104 may have a sharpened end that will assist with penetration of the tip 104 into the tissue of the body 106 during percutaneous entry. The exposed tip 104 represents the active electrode area. The reference electrode 101 typically has a much larger area than the exposed tip 104 so that there is no heating at the surface of the body 106 where the reference electrode 101 is attached. The passage of high frequency electrical current through the needle 102 produces a lesion 105 in the region of the exposed tip 104. The lesion 105 causes coagulation of the neural structures in that region and is responsible for pain relief. It is therefore important to know the position of the exposed tip 104 to gauge the relative position and region that will be affected by the high frequency electrical current.

As stated above, Figure 2 depicts the needle 102 having a hollow shaft typically of one or more metals and a hub 201. Preferably the hub 201 is a Luer lock type molded to the shaft; however, other methods of attachment may be used as will be understood by a person skilled in the art. Inserted in needle 102 through the hollow shaft is an elongate stylet 205 shown in dotted outline. The stylet 205 is adapted to assist in piercing the skin and tissue for entry to a treatment area. The stylet 205 comprises a cap 200 cooperating with Luer lock hub 201. The hub 201 is also operable to accommodate an electrical probe 110 that is inserted into the shaft of the needle 102 when the stylet 205 is removed. A portion of the shaft is covered with an electrically insulating coating 103 leaving the tip 104 exposed. The end-point of the insulating coating 103 on the needle 102 is indicated at numeral 204. In use, the needle 102 with the stylet 205 is inserted into the body 106. Once a correct position has been attained the stylet 205 is removed and the electrical probe 110 that delivers the high frequency electrical current is inserted through the needle 102.

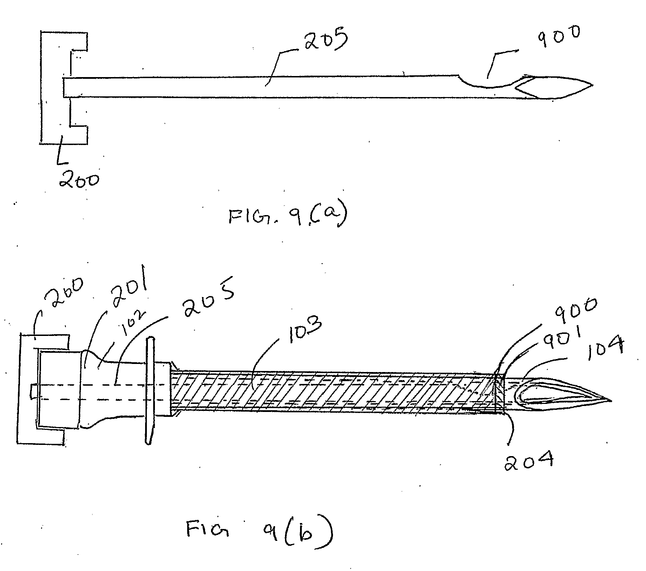

The needle 102 with the stylet 205 is inserted into the patient's body 106 under X-ray/fluoroscopic guidance. One common method for inserting the needle 102 is to locate an X-ray source along one or more desired axes. An image detector on the opposite side of the body portion 106 where the needle 102 is inserted receives the X-rays, thereby permitting verification of the proper location and orientation of the tip 104. Radiopaque marking on the needle 102 or stylet 205 will not only enable its better visualization in this process, but will also indicate the precise location 204 of the end-point of the insulating coating 103 on the needle 102. When the radiopaque marking is on needle 102, the verification of location 204 can be performed along with the verification of the location and orientation of tip 104 by removing the stylet 205 and taking a X-ray/fluoroscopic picture. With reference to Figs. 9A and 9B, radiopaque marking on the stylet 205 may be enhanced by reducing a mass of the stylet 205 at a location 900 of the stylet 205 where the radiopaque band 901 is to be applied. The mass may be reduced by removing material from the stylet 205 such as by grinding or other means. Alternatively the stylet 205 may be originally formed with a reduced mass about the location 900. Radiopaque marking on the stylet 205 may be coupled with grinding of the stylet 205 mass to increase the thickness of the radiopaque band. Alternatively, the needle 102 with the stylet 205 having reduced mass at location 900 could be used to impart radiopacity without any additional band. The stylet 205 could also be made of materials of lower radiopacity than the needle 102 to impart greater illumination.

A radiopaque marker could be applied on selected portions of the needle 102 by, for example, use of masks.

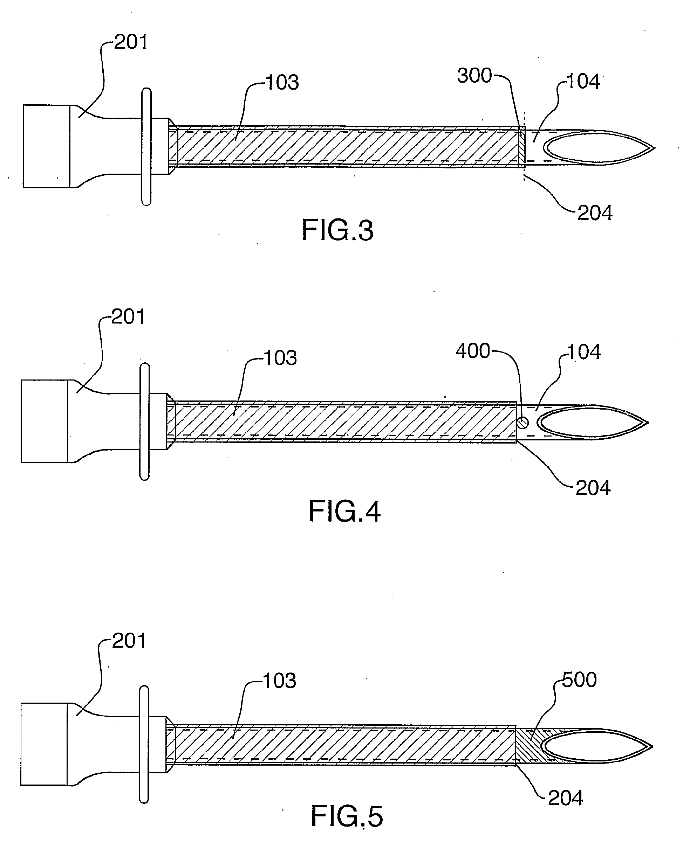

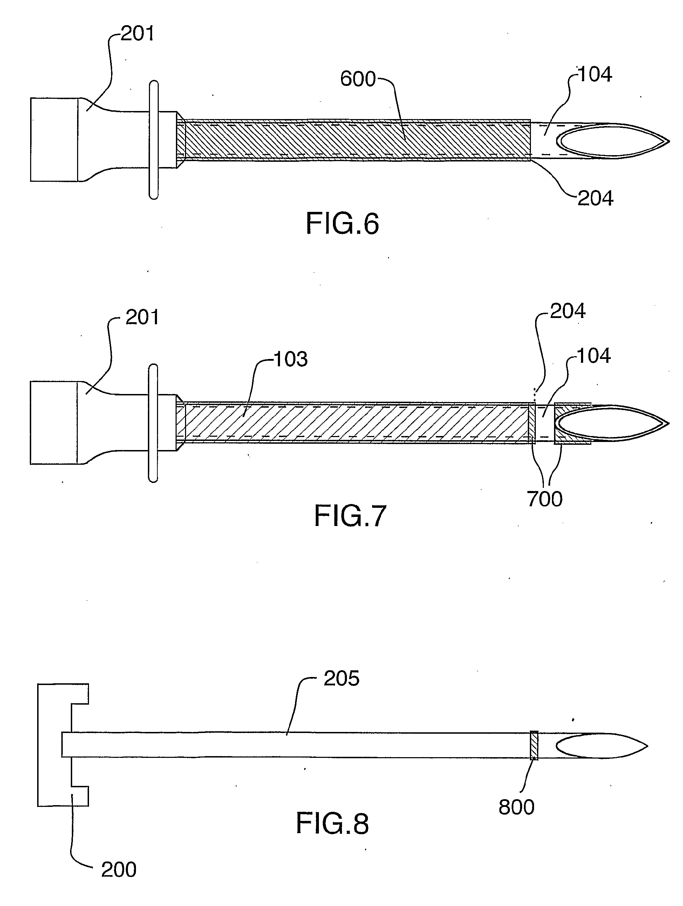

Advantageously selected patterns of radiopacity will allow the precise orientation to be discerned by inspection of the fluoroscopic image. Figures 3 and 7 illustrate different exemplary embodiments of patterns of radiopacity that can be adopted in this invention.

In accordance with the invention, as in the embodiment illustrated in Figure 3, a radiopaque band 300 is located at the edge 204 of the coating and thereby aids in distinguishing between the coated region 103 and uncoated region 104. The radiopaque band 300 is located before the coating end-point 204. It may run 360° around the shaft or be applied through a certain distance of the circumference, for example through 180° or 90°. Figure 3 illustrates one embodiment that includes a radiopaque band 300 through 180° of the shaft, on the side of the beveled tip, just before the coating end-point 204. This provides a clear demarcation between the coated 103 and exposed regions 104 of the needle 102.

The band 300 can be applied in a number of ways including techniques such as, but not limited to, vapor deposition, ion implantation, dip coating, metal plating and electro plating. Bands of radiopaque materials such as platinum iridium bands can also be fused onto the needle 102.

An alternate configuration outside the scope of the invention is depicted in Figure 4. A radiopaque marker 400 may be placed on the needle 102 to distinguish between the coated metal shaft 103 and the exposed metal tip 104 and may be a variety of shapes and sizes. The shape of the marker 400 may also be used to indicate the direction of the beveled tip.

Figure 5 illustrates another configuration outside the scope of the invention. The entire exposed part 104 of the needle 102, is radiopaque indicated at numeral 500 and can be discerned better when viewed under a fluoroscope. The coated region of the needle 103 can be masked and the exposed tip 104 coated with a radiopaque material. Techniques such as vapor deposition and ion bombardment can be used to achieve such coating.

An alternate configuration which is, *per se,* outside the scope of the present invention, can be obtained by imparting radiopacity to the insulating coating 600 as illustrated in Figure 6. The insulating coating on the needle can be made radiopaque in a number of ways such as vapor deposition, ion-bombardment and ion-implantation. This renders the entire insulated portion of the needle radiopaque.

Figure 7 illustrates the needle 102 with two radiopaque bands 700 at the coating end-point 204 and the edge of the exposed tip 104. This defines the region of the exposed tip 104 where delivery of high frequency electrical current to the tissue 106 occurs.

Figure 8 illustrates the stylet 205 with radiopaque marking 800, which may include any of the embodiments described in Figures 3-7 above. The stylet 205 and needle 102 are inserted into the patient's body 106 to ensure correct placement. The radiopacity on the stylet 205 will serve to identify the exposed tip 104 on the needle 102.

An example of suitable material that may be used to impart the desired radiopacity is radiopaque ink with tungsten that is pad printed. The material is selected based on its radiopacity. Addition of a silver solder band is another technique that may be used to accomplish radiopacity. Other suitable materials include, but are not limited to, high-density metals such as platinum, iridium, golf, silver, tantalum or their alloys, or radiopaque polymeric compounds. Such materials are highly visible under fluoroscopic illumination and are therefore visible even at minimal thickness.

The embodiments of the invention described above are intended to be exemplary only. The scope of the invention is therefore intended to be limited solely by the scope of the appended claims.

## Claims

1. A needle (102) for insertion into a patient's body for delivering high frequency electrical current comprising:
an electrically insulated shaft having an electrically insulated portion and an exposed electrically conductive tip portion (104), wherein said insulated portion comprises an insulating coating (103); and
a band-shaped radiopaque marker (300);
**characterised in that** said marker (300) is located at an edge (204) of the coating, to distinguish between the exposed tip portion of the needle (104) and the electrically insulated portion (103) of the needle under fluoroscopic visualization.

2. The needle of claim 1, wherein said insulating coating (103) covers the radiopaque marker (300).

3. The needle of claim 1, wherein the shaft is hollow.

4. The needle of any preceding claim, wherein the needle is operable to connect to an energy source.

5. An apparatus comprising:
the needle of claim 1, wherein the shaft is hollow, the apparatus further comprising:
a stylet (205) operable to be located within the shaft during insertion of said shaft into said patient's body and operable to be removed therefrom after insertion.

6. The apparatus of claim 5, further comprising a probe (110), operable to be connected to an energy source, and operable to be located within said shaft for delivery of high frequency electrical current.

## Patentansprüche

1. Eine Nadel (102) zum Einsetzen in einen Körper eines Patienten zum Liefern von hochfrequentem elektrischen Strom umfassend:
einen elektrisch isolierten Schaft mit einem elektrisch isolierten Bereich und einem elektrisch leitfähigen Spitzenbereich (104), wobei der genannte elektrisch isolierte Bereich eine isolierende Beschichtung (103) aufweist; und
einen bandförmigen strahlenundurchlässigen Marker (300); **dadurch gekennzeichnet, dass** der genannte Marker (300) an einem Rand (204) der Beschichtung vor dem Beschichtungsendpunkt (204) angeordnet ist, um zwischen dem freiliegenden Spitzenbereich der Nadel (104) und dem elektrisch isolierten Bereich (103) der Nadel unter fluoroskopischer Visualisierung zu unterscheiden.

2. Die Nadel nach Anspruch 1, wobei die genannte isolierende Beschichtung (103) den strahlenundurchlässigen Marker (300) bedeckt.

3. Die Nadel nach Anspruch 1, wobei der Schaft hohl ist.

4. Die Nadel nach einem der vorhergehenden Ansprüche, wobei die Nadel betreibbar ist, zu einer Energiequelle zu verbinden.

5. Eine Vorrichtung umfassend:
die Nadel nach Anspruch 1, wobei der Schaft hohl ist, die Vorrichtung weiterhin umfassend:
ein Stilett (205) betreibbar in dem Schaft angeordnet zu sein während des Einsetzens des genannten Schaftes in den genannten Körper des Patienten und betreibbar von diesem entfernt zu werden nach dem Einsetzen.

6. Die Vorrichtung nach Anspruch 5, weiterhin umfassend eine Sonde (110), betreibbar mit einer Energiequelle verbunden zu werden, und betreibbar in dem genannten Schaft angeordnet zu sein zum Liefern von hochfrequentem elektrischen Strom.

## Revendications

1. Aiguille (102) pour insertion dans le corps d'un patient afin d'y délivrer un courant électrique de fréquence élevée, comprenant :
une tige isolée de l'électricité ayant une partie électriquement isolée et une partie de pointe conductrice de l'électricité exposée (104), dans laquelle ladite partie isolée comprend un revêtement isolant (103) ; et
un marqueur radio-opaque en forme de bande (300) ;
**caractérisée en ce que** ledit marqueur (300) est situé sur un bord (204) du revêtement avant le point final (204) du revêtement pour faire la distinction entre la partie de pointe exposée de l'aiguille (104) et la partie électriquement isolée (103) de l'aiguille sous visualisation radioscopique.

2. Aiguille selon la revendication 1, dans laquelle ledit revêtement isolant (103) recouvre le marqueur radio-opaque (300).

3. Aiguille selon la revendication 1, dans laquelle la tige est creuse.

4. Aiguille selon l'une quelconque des revendications précédentes, dans laquelle l'aiguille est à même d'être connectée à une source d'énergie.

5. Appareil comprenant :
l'aiguille selon la revendication 1, dans laquelle la tige est creuse, l'appareil comprenant en outre :
un stylet (205) qui est à même d'être situé dans la tige au cours de l'insertion de ladite tige dans ledit corps du patient et d'en être retiré après insertion.

6. Appareil selon la revendication 5, comprenant en outre une sonde (110) qui est à même d'être connectée à une source d'énergie et d'être située dans ladite tige pour y délivrer un courant électrique de fréquence élevée.
